# EUROPEAN PATENT APPLICATION

(11) **EP 1 550 654 A1**
(43) Date of publication of application: **06.07.2005**
(21) Application number: 04030936.1
(22) Date of filing: 29.12.2004
(51) Int. Cl.: C07C 381/12, G03F 7/029, C08G 59/68

(54) **Aryl sulfonium salt, polymerizable composition and polymerization method of the same**

(30) Priority: 29.12.2003 US 745639
(71) Applicant: Chitec Technology Co., Ltd., Chung Ho Taippei Hsien (TW)
(72) Inventor: Chiu, Chingfan c/o Chitec Technology Co., Ltd., Chung Ho, Taipei Hsien (TW); Yang, Chun Tzu c/o Chitec Technology Co., Ltd., Chung Ho, Taipei Hsien (TW)
(74) Representative: Flaccus, Rolf-Dieter, Dr.

(57) **Abstract**

An aryl sulfonium salt, whose photo-induced products are non-benzene, is represented by the formula (I): 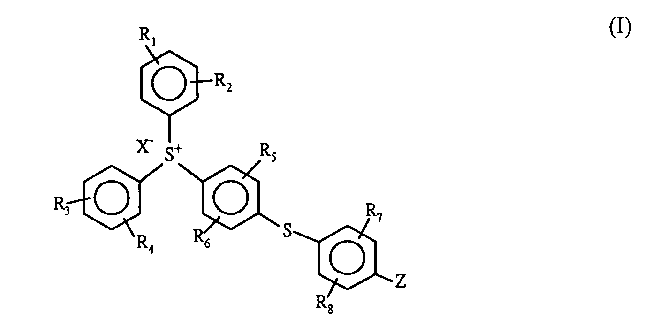 wherein Z represents a hydrogen atom or the formula (II):

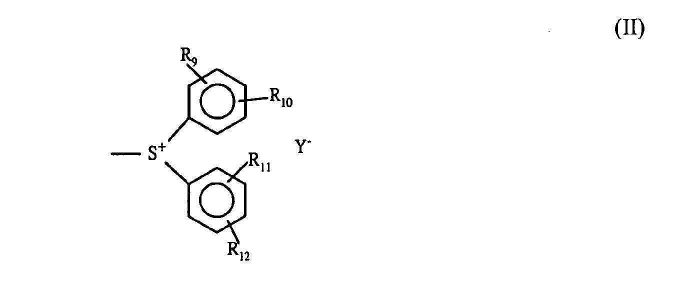

The substituted groups of R₁ to R₁₂ are independently a hydrogen atom, a C₁ to C₁₂ alkyl group or a C₃ to C₁₂ cyclic alkyl group, and each phenyl group independently has at least one of the alkyl group or the cyclic alkyl group; and X⁻ and Y⁻ are independently an anion. A polymerizable composition containing the aryl sulfonium salt and a polymerization method of the same are provided.

## Description

### Field of the Invention

Generally, the present invention relates to an aryl sulfonium salts, a radiation polymerizable composition containing the aryl sulfonium salts, and the polymerization method of the same.

### Background of the Invention

Aryl sulfonium salts, which extensively applied in coatings, adhesives, inks, photo resists, or 3D Stereolithography have been used in industries for many years. Due to its highly photo-active property, aryl sulfonium salts can be a photochemical source of strong acid to initiate polymerization of the monomers, dimmers, oligomers and related polymers. The commercialization of aryl sulfonium salts as photoinitiators in photo-polymerization industry and as photoacid generators in lithographic technology has been proven to be very successful.

However, recently aryl sulfonium salts are limited to be used because the suspected carcinogen of benzene has been detected in the photocuring process as the byproduct. Since the unpopularity of benzene in benzene-releasing aryl sulfonium salts may deter the acceptances of the aryl sulfonium salts, the needs for finding a "benzene-free" substitute for new aryl sulfonium salts have arisen.

Haker et al ("Photochemistry of Triarylsulfonium Salts", *J*. *Am. Chem. Soc.* **1990**, *112,* 6004-6015) and Turro et al ("Photo-CIDNP and Nanosecond Laser Flash Photolysis Studies on the Photodecomposition of Triarylsulfonium Salts", *J Org. Chem*. **1992**, *57,* 4179-4184) had reported the irradiation mechanisms of aryl sulfonium salts. Based on their report, benzene is released from the cleavage of the phenyl-sulfur bond during the aryl sulfonium salts are irradiated by ultraviolet light. It is also noted that toluene could be generated instead of benzene in irradiation of an aryl sulfonium salt having a methyl group attached to the phenyl group adjacent to the corresponding sulfur atom.

Although some currently used aryl sulfonium salts of low molecular weight, such as tris (4-methylphenyl) sulfonium salts, tris(4-chlorophenyl) sulfonium salt, are able to generate the residues without benzene during irradiation, they cannot absorb the longer wavelength ultraviolet light and produce colorless products. Some patents have focused on distinctive synthesis methods of aryl sulfonium salts and U.S. Pat. No. 2,807,648 disclosed one conventional approach to synthesize the aryl sulfonium salts in 1957; however, the "benzene-free" issue has not been addressed until recently.

Therefore, there has been continued need for the aryl sulfonium salts with benzene-free photo generation property as well as relatively broad spectral absorbency to meet both application and environmental needs.

### Summary of the Invention

The present invention provides an aryl sulfonium salt having at least one of alkyl groups or cyclic alkyl groups attached to each phenyl group adjacent to the corresponding sulfur atom, a radiation polymerizable composition containing the aryl sulfonium salt and the method to polymerize the radiation polymerizable composition. Irradiation of the aryl sulfonium salt avoids generating the suspected carcinogen of benzene.

The aryl sulfonium salt is represented by the formula (I): 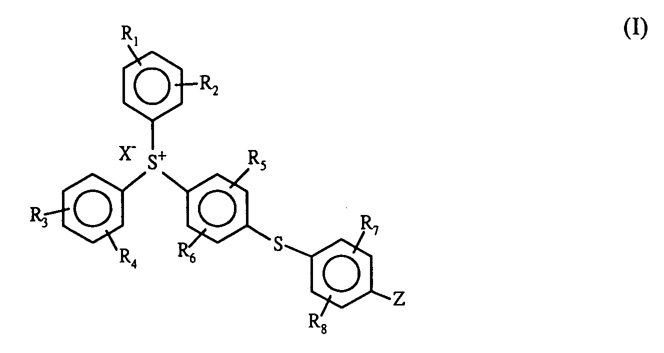 wherein Z represents a hydrogen atom or the formula (II): 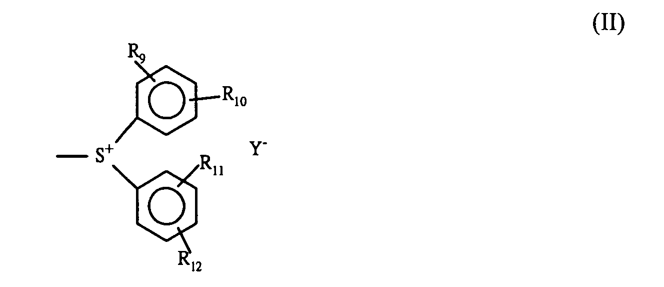 the substituted groups of R₁ to R₁₂ are independently selected from the group consisting of a hydrogen atom, a straight or a branched C₁ to C₁₂ alkyl group, and a C₃ to C₁₂ cyclic alkyl group;
each phenyl group independently has at least one of the substituted groups selected from the group consisting of said alkyl group and said cyclic alkyl group;
X⁻ and Y⁻ independently represent an anion.

### Detailed Description of the Invention

The idea in this invention is that if each phenyl group of aryl sulfonium salts owns at least one of methyl, any other alkyl group, or cyclic alkyl group, the residues during irradiation would be toluene, any other alkyl benzene or cyclic alkyl benzene, rather than the highly toxic benzene. Accordingly, the following elaborations focus on an aryl sulfonium salt whose photo-induced products are benzene-free, a radiation polymerizable composition containing the benzene-free aryl sulfonium salt and a polymerization method using the radiation polymerizable composition.

### (a) aryl sulfonium salt and its synthesis

The aryl sulfonium salt is represented by the formula (I): 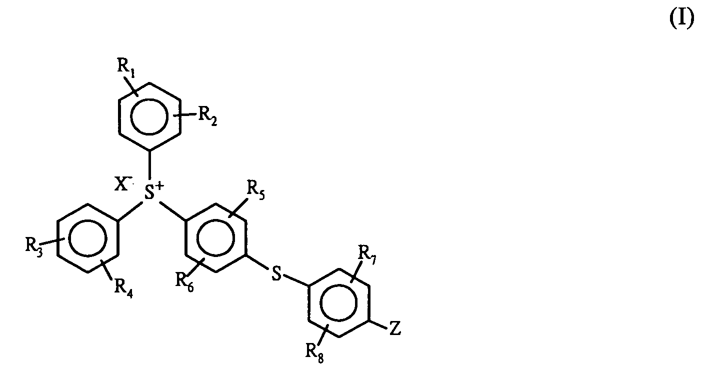 wherein Z represents a hydrogen atom or the formula (II): 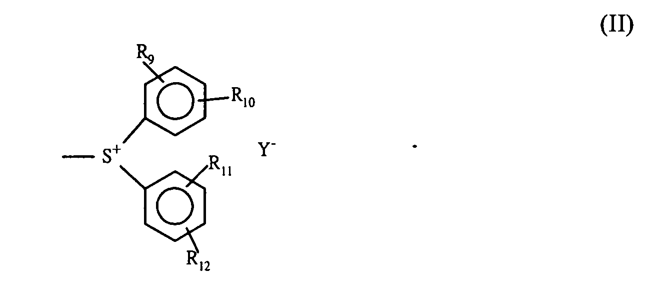 the substituted groups of R₁ to R₁₂ are a hydrogen atom, a straight or a branched C₁ to C₁₂ alkyl group, or a C₃ to C₁₂ cyclic alkyl group; each phenyl group independently has at least one of the substituted groups selected from the group consisting of the alkyl group and the cyclic alkyl group; X⁻ and Y⁻ independently represent an anion.

The substituted groups of R₁ to R₁₂ in the formula (I) and (II) are independently preferable to be a hydrogen, or an alkyl group such as methyl, ethyl, propyl, isopropyl, butyl, isobutyl, t-butyl, decyl, or a cyclic alkyl group such as cyclopropyl, cyclopentyl, cyclohexyl, etc. Any of the substituted groups may be at ortho, meta or para directed relative to the carbon-sulfur. Besides, any two of the alkyl groups adjacent to the same phenyl group may be bonded to each other to form an alkylene bridge, thereby, the structure may have a ring in the formula (I) or (II). At all events, each phenyl group attached to the sulfur atom of the formula (I) and (II) independently has at least one of the alkyl groups or cyclic alkyl groups to function as a non-benzene generation property in photodecomposition. Further, X⁻ and Y⁻ are independently an anion. Preferred examples include SbF₆⁻, PF₆⁻, AsF₆⁻, BF₄⁻, B(C₆F₅)₄⁻, CF₃SO₃⁻, ClO₄⁻ or FSO₃⁻ but are not limited thereto.

The manufacture procedures include forming a mixture by mixing a reactant with aluminum chloride, and then subsequently reacting the mixture with sulfur mono chloride and chlorine gas to produce a mixture of aryl sulfonium chloride. The preferred reactant used is selected from a straight or a branched alkyl benzene, such as toluene, t-butyl benzene, or cyclic alkyl benzene such as cyclobutyl benzene, cyclopentyl benzene, cyclohexyl benzene, cycloheptyl benzene, cyclopropylmethyl benzene, 1,2,3,4-tetrahydronaphthalene, etc. Further, in the reaction step with sulfur mono chloride, the present invention lasts for one hour long or more until the sulfur mono chloride has been completely reacted before chlorine gas addition. Continually, as the aryl sulfonium chloride is produced, a solution containing a reactant having an anion such as SbF₆⁻, PF₆⁻, AsF₆⁻, BF₄⁻, B(C₆F₅)₄⁻CF₃SO₃⁻, ClO₄⁻ or FSO₃⁻ etc. is added to react. Then the precipitate of the aryl sulfonium salt is found which can be stored in its neat form or dissolved in a solvent. The solvent includes alkyl carbonate, lactams, lactones or ketones, which is preferred propylene carbonate and caprolactone.

Toluene and PF₆⁻ are the most preferable reactants to synthesize the aryl sulfonium salts of this present invention. Other details of experiments will be described in following practical examples.

### (b) radiation polymerizable composition containing the aryl sulfonium salt

The radiation polymerizable composition consists of the aryl sulfonium salts of this present invention and cationically polymerizable monomers. In addition to that, other monomers or additives such as stabilizer, pigments, or surfactants can be included for some specific purposes, which those of ordinary skill in the art may prefer.

The cationically polymerizable monomers in this present invention include acid labile moieties which is structured as, for examples, vinyl ester monomers, epoxy resins, ketones, lactones, oxetanes, acroleins, spiro-orthocarbonates, spiro-orthoester, or phenol formaldehyde resins, etc. The most preferable among them are epoxy resins and vinyl esters monomers.

Typical vinyl esters monomers include alkyl vinyl ether compounds, such as methyl vinyl ether, isobutyl vinyl ether, aryl vinyl ether such as phenyl vinyl ether, p-methoxyphenyl vinyl ether, which contains at least one ethylentically unsaturated group. Typical examples of epoxy resins include the epoxidized novolak polymers, the polyepoxides from halo-epoxy alkanes such as epichlorohydrin and a polynuclear dihydric phenol such as bisphenol A. Mixture of those epoxides can be used when desired. In this radiation polymerizable composition, the aryl sulfonium salt represented by formula (I) and (II) is about 0.1% to 10% by weight of the monomers therein and preferably 0.6 % to 5 %. The ratio is determined according to the type, dosage of electromagnetic radiation, and other factors such as desired cure time, temperature, humidity, or coating thickness.

### (c) polymerization method using the polymerizable composition

The radiation polymerizable composition containing the aryl sulfonium salts represented by formula (I) and (II) can be polymerized under exposed under electromagnetic radiation. Generally, the composition of this invention is a liquid having a viscosity of 1 to 100,000 centipoise at 25°C or a solid dissolved in a suitable solvent. Under electromagnetic radiation within from a tenth of minute to several minutes, it becomes dry to touch (usually known as "tack free") or solvent-insoluble as the intensity of the ultraviolet light is sufficient to induce the reactivity of the aryl sulfonium salts. The preferred electromagnetic radiation wavelength is between 200 nm and 500 nm, and particularly ultraviolet radiation is most preferable. Therefore, low-pressure mercury lamps, medium-pressure mercury lamps, high-pressure mercury lamps, xenon lamps, carbon arc lamps, and the like are candidates of the electromagnetic radiation sources.

### (d) Determination of residue benzene in cationically radiation polymerization

The determination of residue benzene in cationically radiation polymerization is performed by an automated headspace gas chromatograph equipped with a flame ionization detector having helium as a carrier gas. The prepared sample is weighed into a headspace vial, sealed and allowed to equilibrate at 150°C for 30 minutes to release benzene from the solid. The time and temperature mentioned have been found sufficient to equilibrate benzene between solid and gas phases. The sample is spiked using a benzene/methanol solution at three different levels and then a standard addition calculation is performed to quantify benzene in the sample.

The present invention has some preferred examples shown as below.

### Example 1

210 g (1.6mole) aluminum chloride is added into a 500ml, three-neck, roundbottomed flask charged with 580 g toluene. The solution is stirred and cooled to about 10 °C. Then 100g (0.74 mole) sulfur monochloride is added slowly into the solution, keeping the temperature between 13°C-18°C. After about one-hour reaction, 200g chlorine is then bubbled into the solution while keeping the temperature between 13°C-18°C. Following this addition, the solution is poured into 600g ices placed in a flask with stirring until the entire solid has been dissolved. Then the solution is settled for 30 minutes to separate the lower product layer, which is then extracted with sulfuric acid solution (50 g H₂SO₄ in 600 water) and sodium hydroxide solution (20g NaOH in 100 g water) in sequence. The lower product layer obtained by extraction is then poured into 600ml water placed by another flask. After that, a solution containing KPF₆ (110 g KPF6 in 400 ml water) is added into the mixture and stirred then the participated is found. The yield is 340g, 80% yield rate after filtration from the solution. The analysis results : melting point: 98°C-112°C, Mass Spectrum: m/z : 427(100%), 785(35%), 305(20%), 639(5%).

### Example 2

121 g (0.89 mole) aluminum chloride is added into a 500ml, three-neck, roundbottomed flask charged with 298g n-Butyl benzene. The solution is stirred and cooled to about 10°C. Then 50 g ( 0.37mole) sulfur monochloride is added slowly into the solution, keeping the temperature between 13°C-18°C. After about one-hour reaction, 46 g chlorine is then bubbled into the solution while keeping the temperature between 13°C-18°C. Following this addition, the solution is poured into 300g ices placed in a flask with stirring until the entire solid has been dissolved. Then the solution is settled for 30 minutes to separate the lower product layer, which is then extracted with sulfuric acid solution (25 g H₂SO₄ in 300 water) and sodium hydroxide solution (10g NaOH in 50 g water) in sequence. The lower product layer obtained by extraction is then poured into 300ml water placed by another flask. After that, a solution containing KPF₆ (55 g KPF6 in 200 ml water) is added into the mixture and stirred then the participated is found. The yield is 203.1 g, 75 % yield rate after filtration from the solution. The analysis result: Mass Spectrum m/z: 543.33(100%), 595.27(52%), 431.27(23%), 1037.20(9%)

### Example 3

121g (0.89mole) aluminum chloride is added into a 500ml, three-neck, roundbottomed flask charged with 500g 1,2,3,4-tetrahydronaphthalene. The solution is stirred and cooled to about 15°C. Then 50 g ( 0.37 mole) sulfur monochloride is added slowly into the solution, keeping the temperature between 20°C-25°C. After about one and half-hour reaction, 60g chlorine is then bubbled into the solution while keeping the temperature between 20°C -25°C. Following this addition, the solution is poured into 300 g ice placed in a flask with stirring until the entire solid has been dissolved. Then the solution is settled for 30 minutes to separate the lower product layer, which is then extracted with sulfuric acid solution (30 g H₂SO₄ in 360 water) and sodium hydroxide solution (10g NaOH in 50 g water) in sequence. The lower product layer obtained by extraction is then poured into 300ml water placed by another flask. After that, a solution containing KPF₆ (55 g KPF6 in 200 ml water) is added into the mixture and stirred then the participated is found. The yield is 161.2 g, 66% yield rate after filtration from the solution. The analysis result: Mass Spectrum m/z : 425.27(100%), 426.27(33%), 479.27(18%), 587.13(3%).

### Test Example

### (1) Photo Curing Test

An aryl sulfonium salt is dissolved in propylene carbonate to prepare a 40wt% solution. Then 0.1g the solution is mixed with 1.8g EB 1500 (Epoxy monomers from UCB), 0.2g Tone-0301 (Polyol from DOW) and 0.004g L7604 (surfactant form Witco) to form a composition. The composition is then applied onto a aluminum plate to form a 3 µ m thickness film and the film is allowed to polymerize under following conditions:

UV Irradiation Apparatus:

Lamp: Fusion F300 series, 120W/cm (80 w/inch) D bulb

Conveyer speed: 60 m/min;

Time of irradiation: 1 s

(2) Benzene Residue Test

Weigh 0.08g-0.12g cured polymers into a 20ml headspace cap vial. Crimp the vial and heat the cured polymer at 150°C for 30 minutes. Analyze the cured polymer using the headspace gas chromatography under following conditions:

Perkin-Elmer AutoSystem GC interfaced with HS-40 Headspace Sampler or equivalent equipped with a flame-ionization detector;

Capillary column:30m x 0.25 ID mm x 1.0 µm film thickness J&W DB-5;

Carrier Gas: Helium 27 psi headpressure;

Oven Temperature: 30°C for 3 min then 5°C /min to 75°C then 25°C/min to 260°C hold for 8 min;

Split Vent: 44 ml/minute;

Inlet Temperature.: 240°C;

Detector Temperature: 280°C.

The test results are shown in Table 1.

**Table 1**

| Sulfonium salt | Weight | Photo cuing | Benzene residue |
|---|---|---|---|
| Compound in Example 1 | 0.1g | + | <0.1ppm |
| Compound in Example 2 | 0.1g | + | <0.1ppm |
| Compound in Example 3 | 0.1g | + | <0.1ppm |
| UVI 6992 (Comparative) | 0.08g | + | 50ppm |
| (1) UVI 6992: 50 % solid content from DOW (2) "+": tack-free; "-": remaining tack. | | | |

## Claims

1. An aryl sulfonium salt represented by the formula (I): 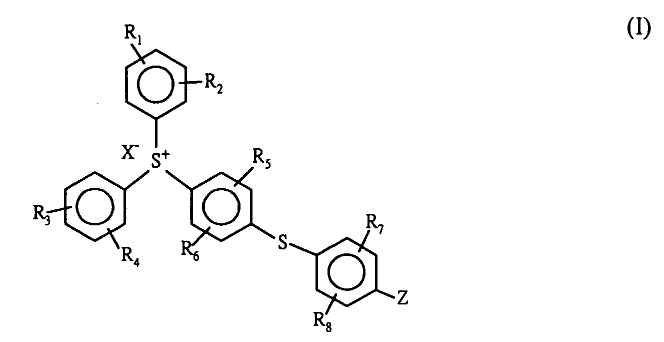 wherein Z represents a hydrogen atom or the formula (II): 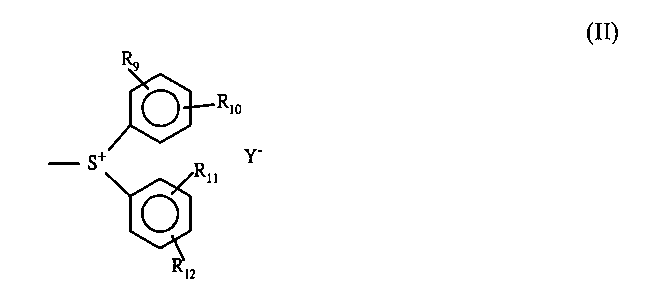 the substituted groups of R₁ to R₁₂ are independently selected from the group consisting of a hydrogen atom, a straight or a branched C₁ to C₁₂ alkyl group, and a C₃ to C₁₂ cyclic alkyl group;
each phenyl group independently has at least one of the substituted groups selected from the group consisting of said alkyl group and said cyclic alkyl group;
X⁻ and Y⁻ independently represent an anion.

2. The aryl sulfonium salt of claim 1, wherein said anion is selected from the group consisting of SbF₆⁻, PF₆⁻, AsF₆⁻, BF₄⁻, B(C₆F₅)₄⁻, CF₃SO₃⁻, ClO₄⁻ and FSO₃⁻.

3. The aryl sulfonium salt of claim 1, wherein said substituted groups comprise an alkylene group formed by bonding two of said alkyl groups adjacent to the same phenyl group.

4. The aryl sulfonium salt of claim1, wherein said alkyl group comprises a methyl group.

5. The aryl sulfonium salt of claim1, wherein said aryl sulfonium salt is in its neat form or dissolved in a solvent.

6. The aryl sulfonium salt of claim 5, wherein said solvent selected from the group consisting of alkyl carbonates lactams, ketones and lactones.

7. An aryl sulfonium salt represented by the formula (I): 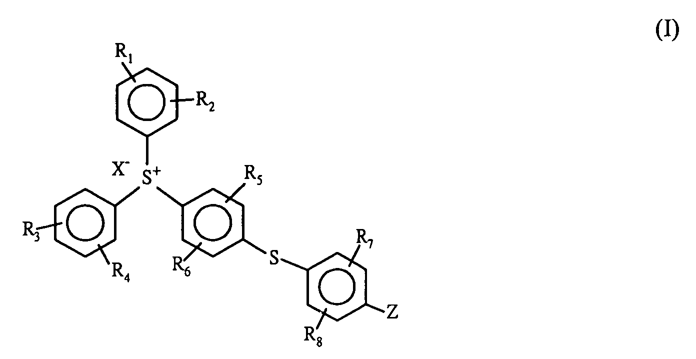 wherein
Z represents a hydrogen atom or the formula (II): 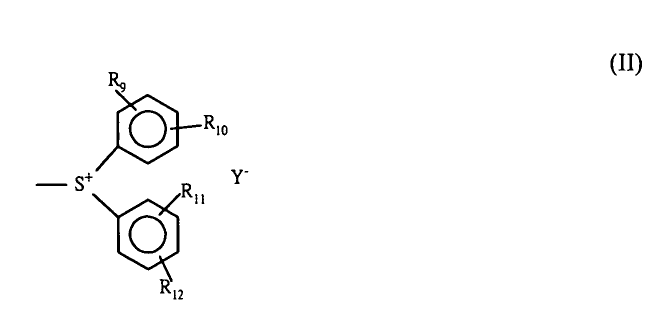 the substituted groups of R₁ to R₁₂ are independently selected from the group consisting of a hydrogen atom, a straight or a branched C₁ to C₁₂ alkyl group, and a C₃ to C₁₂ cyclic alkyl group;
each phenyl group independently has at least one of the substituted groups selected from the group consisting of said alkyl group and said cyclic alkyl group;
X⁻ and Y⁻ independently represents an anion selected from the group consisting of SbF₆⁻, PF₆⁻, AsF₆⁻, BF₄⁻, B (C₆F₅)₄⁻, CF₃SO₃⁻, ClO₄⁻ and FSO₃⁻.

8. The aryl sulfonium salt of claim 7, wherein said substituted groups comprise an alkylene group formed by bonding two of said alkyl groups adjacent to the same phenyl group.

9. The aryl sulfonium salt of claim 7, wherein said alkyl group comprises a methyl group.

10. The aryl sulfonium salt of claim 7, wherein said aryl sulfonium salt is in its neat form or dissolved in a solvent.

11. The aryl sulfonium salt of claim 10, wherein said solvent selected from the group consisting of alkyl carbonates or lactones.

12. A radiation polymerizable composition comprising a cationically polymerizable monomer and an aryl sulfonium salt represented by the formula (1): 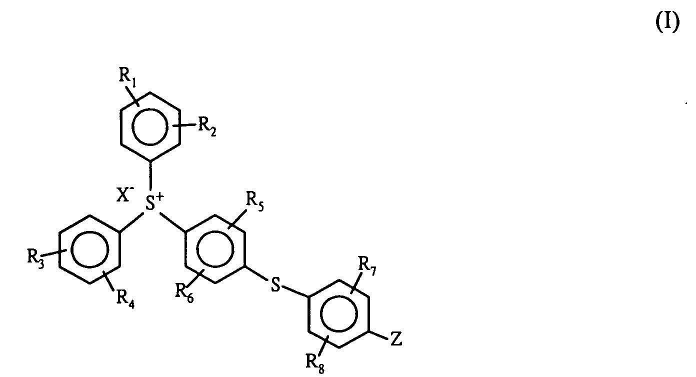 wherein
Z represents a hydrogen atom or the formula (II): 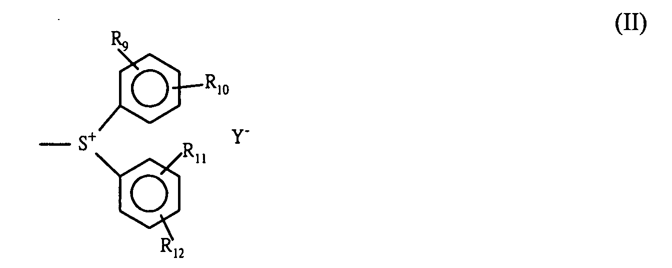 the substituted groups of R₁ to R₁₂ are independently selected from the group consisting of a hydrogen atom, a straight or a branched C₁ to C₁₂ alkyl group, and a C₃ to C₁₂ cyclic alkyl group;
each phenyl group independently has at least one of the substituted groups selected from the group consisting of said alkyl group and said cyclic alkyl group;
X⁻ and Y⁻ independently represents an anion.

13. The composition of claim 12, wherein said anion is selected from the group consisting of SbF6⁻, PF₆⁻, AsF₆⁻, BF₄⁻, B (C₆F₅) 4⁻, CF₃SO₃⁻, ClO₄⁻ and FSO₃⁻.

14. The composition of claim 12, wherein said cationically polymerizable monomer comprises an epoxide group.

15. The composition of claim 12, wherein said cationically polymerizable monomer comprises an ethylentically unsaturated group.

16. The composition of claim 12, wherein said aryl sulfonium salt is about 0.1 % to about 10% by weight of the monomers therein.

17. The composition of claim 12, wherein said substituted groups comprise an alkylene group formed by bonding two of said alkyl groups adjacent to the same phenyl group.

18. The composition of claim 12, wherein said alkyl group comprises a methyl group.

19. A method of polymerization of a radiation polymerizable composition wherein the steps comprises:
(a) forming a radiation polymerizable composition by mixing cationically polymerizable monomers with an aryl sulfonium salt represented by the formula (I): 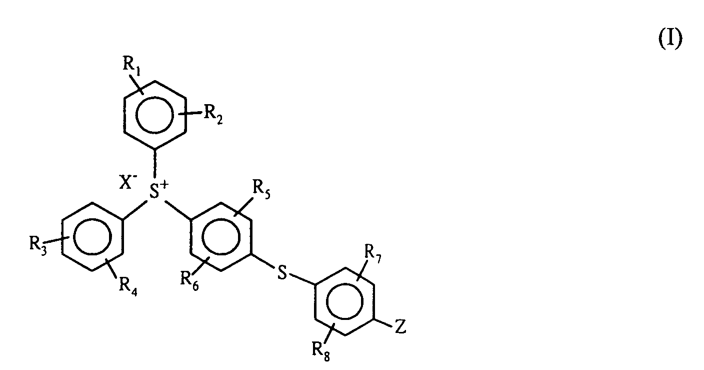 Wherein Z represents a hydrogen atom or the formula (II): 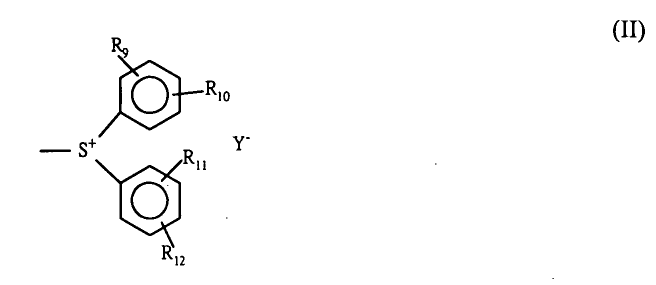 the substituted groups of R₁ to R₁₂ are independently selected from the group consisting of a hydrogen atom, a straight or a branched C₁ to C₁₂ alkyl group, and a C₃ to C₁₂ cyclic alkyl group; each phenyl group independently has at least one of the substituted groups selected from the group consisting of said alkyl group and said cyclic alkyl group;
X⁻ and Y⁻ independently represent an anion;
(b) exposing said radiation polymerizable composition to electromagnetic radiation sufficiently to initiate polymerization of said radiation polymerizable composition.

20. The method of claim 19, wherein said electromagnetic radiation having a wavelength of about 200nm to about 500nm.

21. The method of claim 19, wherein said anion is selected from the group consisting of SbF₆⁻, PF₆⁻, AsF₆⁻, BF₄⁻, B (C₆F₅)₄⁻, CF₃SO₃⁻, ClO₄⁻ and FSO₃⁻.

22. The method of claim 19, wherein said substituted groups comprises an alkylene group formed by bonding two of said alkyl groups adjacent to the same phenyl group.

23. The method of claim 19, wherein said alkyl group comprises a methyl group.

24. The method of claim 19, wherein said cationically polymerizable monomers comprise an acid labile moiety.

25. The method of claim 19, wherein said cationically polymerizable monomers comprise an epoxide group.

26. The method of claim 19, wherein said cationically polymerizable monomers comprise an ethylentically unsaturated group.

27. The method of claim 19, wherein said aryl sulfonium salt is about 0.1 % to 10% by weight of the monomers therein.
